# EUROPEAN PATENT APPLICATION

(11) **EP 3 626 466 A1**
(43) Date of publication of application: **25.03.2020**
(21) Application number: 19198831.0
(22) Date of filing: 23.09.2019
(51) Int. Cl.: B41J 11/00, A61B 90/00, A61B 90/14

(54) **SUPPORT BASE FOR CONVEYOR BELTS IN CONTINUOUS DIGITAL PRINTING MACHINES**

(30) Priority: 24.09.2018 ES 201830919
(71) Applicant: Asitec Ceramic, S.L., 12110 Alcora (ES)
(72) Inventor: BARREDA FERRANDO, Juan José, 12110 ALCORA (ES)
(74) Representative: Sanz-Bermell Martinez, Alejandro

(57) **Abstract**

The invention relates to a glass support base for conveyor belts in continuous digital printing machines, comprising a set of openings, and arranged on a case (2) in which a vacuum is created, causing suction through the openings arranged in the base, and on which a perforated belt (4) moves, conveying pieces (5) through a printing machine (3).

## Description

The present invention belongs to the field of the art of digital printing, and particularly to the conveyor system for the pieces to be printed.

### State of the art

Industrial printing is a process comprising various methods, where printing systems greatly vary.

Among these systems are some such as flexography, offset printing, or rotogravure, all of which are different systems, but in them, the image is transferred from a plate or roller to the piece to be printed. In these systems, the piece to be printed (paper, cardboard, plastic, etc.) is conveyed and guided between a series of rollers.

In addition to the mentioned systems, digital printing has been developed in recent years, where this type of printing allows for greater flexibility since a printing plate is not needed, it can be adapted to different sizes, and it is cost-effective for shorter for print runs. Within digital printing methods there are, among others, ink injection and laser printing. When continuous digital printing is performed, the piece to be printed is moved via a conveyor through the printing machine, where the print heads printing the image on the piece are located. To assure that the image is printed perfectly in the piece, said piece must be held against the surface on which it is moved so as to prevent unwanted movements which change its position before or during printing.

Therefore, in machines of this type using this printing technique, the conveyance of the pieces is performed by means of vacuum conveyor belts, which consist of a perforated belt connected to a low pressure (vacuum) area through openings in the bed of the belt, such that said vacuum generates a suction which "sticks" the conveyed piece to the conveyor belt, thus being able to perform the printing operation such that the element to be printed does not move, and thus achieving the required quality in the printed image.

In the described vacuum conveyors, the perforated belt is arranged on a case with two side walls and a perforated upper base, said base being made of a metallic material, such that a negative pressure (vacuum) is created inside the case, and suction is produced through both the openings of the perforated support base and in the openings of the perforated belt, holding the piece on the belt and preventing the piece from moving with respect to said belt.

A problem which arises in this type of conveyors is that between the metallic base and the perforated belt considerable friction is produced, causing significant wear both in the perforated belt and in the metallic base, which entails high maintenance, since both the metallic base and the perforated belt must be periodically replaced, and therefore productivity is lost because time and manual labor have to be used in replacing them.

To solve this problem, the present invention provides a solution for the vacuum belt conveyors for conveying pieces (paper, cardboard, plastic, etc.) in continuous digital printing machines, solving the aforementioned problems.

### Description of the invention

The present invention provides a substantial improvement in digital printing machine conveyors, in which the piece to be printed is conveyed on a belt comprising a set of uniformly or non-uniformly arranged openings and operated by rollers, and the pieces are held on same by means of a vacuum system.

Usually, in digital printing machine conveyors, the perforated belt is arranged on a case with two side walls and a perforated upper base, such that a vacuum is created inside the case, and a suction is produced through both the openings of the perforated support base and the openings of the perforated belt, holding the piece on the belt and preventing the piece from moving with respect to said belt.

The present invention provides a support base for perforated conveyor belts on which the latter slide, said base being made up of at least one glass plate.

The fact that the support base of the perforated belt is made of glass means that the friction produced between said base and the perforated belt is much lower than the friction produced when the support base is made of metal. This causes both the support base and the perforated belt to experience much less a wear mucho, which considerably reduces the required maintenance, and the energy consumption dissipating such friction is also reduced to some extent. The number of production stoppages for changing the belt and/or support base is thereby reduced, which, in addition to being a difficult job, noticeably reduces productivity.

According to one embodiment of the present invention, the support base is made up of a set of plates which are arranged juxtaposed on the vacuum case longitudinally in the movement direction of the pieces. The fact that the support base is made up of different plates facilitates the transport and assembly operations of the support base, and should one of the plates break, only the damaged plate would have to be replaced, and not the entire support base, particularly if said support base has a significant length.

According to one embodiment of the present invention, the glass is a laminated glass. The advantage with the glass being laminated is that should it crack, it prevents shards of glass from breaking off, considerably improving the mechanical behavior thereof in the event of impact and preventing the danger that the casting off of slivers of glass represents both for operators and for the printing machine.

In order to guarantee that suction is always produced on the piece being conveyed for printing thereof, the openings of the support base are of a size different from the openings of the perforated conveyor belt, such that it is assured at all times that at least one portion of a set of openings of the base coincides with at least one portion of a set of openings of the conveyor belt.

According to one embodiment, the openings of the conveyor belt are of a larger size than the openings of the support base and are arranged such that at least one portion of each opening of the base coincides with at least one portion of an opening of the belt during operation.

According to another embodiment, the openings of the support base are of a larger size than the openings of the conveyor belt and are arranged such that at least one portion of each opening of the base coincides with at least one portion of an opening of the belt during operation.

### Brief description of the drawings

For the purpose of illustration the explanations to follow, a sheet of drawings has been attached to the present specification, wherein two figures depict, by way of non-limiting example, the essence of the present invention according to a particular embodiment, and in which:
- Figure 1: shows the perforated glass base arranged on the conveyor element of a continuous digital printing machine, according to an embodiment of the present invention.
- Figure 2: shows the perforated belt arranged on the perforated glass base in a continuous digital printing machine, according to an embodiment of the present invention.

The following reference signs can be seen in said figures:
- 1: Perforated glass base
- 2: Support case
- 3: Printer
- 4: Perforated belt
- 5: Piece to be printed

### Description of the preferred embodiments of the invention

In view of the mentioned figures, and according to the numbering used, a preferred embodiment of the invention can be observed therein, which comprises the parts and elements indicated and described in detail below.

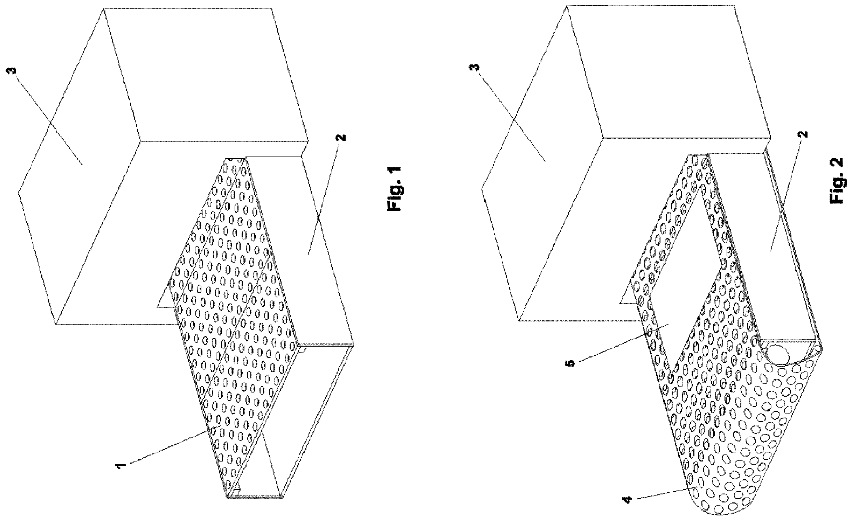

Thus, as can be observed in Figures 1 and 2, the present invention provides a perforated glass base (1) for a vacuum belt conveyor, in which the glass base (1) is arranged on at least two sides plates forming a case (2) inside which a vacuum is created, causing a suction through the openings made in said base (1). A perforated belt (4) operated by a set of rollers travels over the glass base (1), said perforated belt (4) going through the printing machine (5).

The vacuum created inside the case (2) causes a suction through the openings of the base (1) and of the perforated belt (4), which causes the conveyed piece (5) to remain fixed on the perforated belt (4) (which belt slides together with the piece through the printing machine), thus allowing the printing operation to be carried out correctly.

To guarantee that suction is produced the conveyed piece (5) at all times, the openings of the perforated belt (4) are of a larger size than the openings of the glass base (1) and are arranged such that at least one portion of each opening of the glass base (1) coincides with at least one portion of an opening of the perforated belt (4) during operation, or vice versa.

## Claims

1. Support base (1) for conveyor belts in continuous digital printing machines, comprising a set of openings, which is arranged on a case (2) in which a vacuum is created, causing suction through the openings arranged in the base (1), and on which a perforated belt (4) moves, conveying pieces (5) through a printing machine (3), **characterized in that** the support base (1) is made of glass.

2. Support base (1) for conveyor belts in continuous digital printing machines according to claim 1, **characterized in that** the glass making up the support base (1) is a laminated glass.

3. Support base (1) for conveyor belts in continuous digital printing machines according to any of the preceding claims, **characterized in that** the openings of the glass base (1) are of a smaller size than the openings of the perforated belt (4) and are arranged such that at least one portion of each opening of the glass base (1) coincides with at least one portion of an opening of the perforated belt (4) during operation.

4. Support base (1) for conveyor belts in continuous digital printing machines according to any of claims 1 or 2, **characterized in that** the openings of the glass base (1) are of a larger size than the openings of the perforated belt (4) and are arranged such that at least one portion of each opening of the glass base (1) coincides with at least one portion of an opening of the perforated belt (4) during operation.

5. Support base (1) for conveyor belts in continuous digital printing machines according to any of the preceding claims, **characterized in that** the base is made up of different juxtaposed plates.
